**Europäisches Patentamt**

**European Patent Office**

**Office européen des brevets**

(19)

(11) Numéro de publication: **0 196 238**
**B1**

(12) **FASCICULE DE BREVET EUROPEEN**

(45) Date de publication du fascicule du brevet:
16.08.89

(51) Int. Cl.⁴: **A 61 B 6/06**

(21) Numéro de dépôt: **86400156.5**

(22) Date de dépôt: **27.01.86**

(54) Collimateur pour appareils de tomographie.

(30) Priorité: **28.01.85 FR 8501120**

(43) Date de publication de la demande:
**01.10.86 Bulletin 86/40**

(45) Mention de la délivrance du brevet:
**16.08.89 Bulletin 89/33**

(84) Etats contractants désignés:
**AT BE CH DE FR GB IT LI LU NL SE**

(56) Documents cités:
**AT-B- 339 440**
**DE-C- 387 082**
**FR-A- 2 530 035**
**US-A- 3 997 794**
**US-A- 4 087 694**

(73) Titulaire: **MEDICORP RESEARCH LABORATORIES CORPORATION, 1200 North Federal Highway Suite 200-26, Boca Raton Florida 33432 (US)**

(72) Inventeur: **Karcher, Gilles, 2, Rue Lafayette, F-54000 Nancy (FR)**
Inventeur: **Amor, Max, 9, Square de Liège, F-54500 Vandoeuvre (FR)**
Inventeur: **Niddam, Roger, 43, Allée du Jardin Anglais, F-93340 Le Rancy (FR)**
Inventeur: **Villemot, Jean-Pierre, Central Parc 24 Avenue de l'Armée Patton, F-54000 Nancy (FR)**

(74) Mandataire: **Laget, Jean-Loup et al, Cabinet Pierre Loyer 77, rue Boissière, F-75116 Paris (FR)**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

**Description**

L'invention concerne un collimateur pour tomoscintigraphie. La tomoscintigraphie est un procédé d'examen utilisé en médecine nucléaire, dans lequel on enregistre des images scintigraphiques d'un organe sous de nombreuses incidences, et on reconstruit des coupes transverses de cet organe au moyen d'un système informatique.

Pour l'enregistrement d'images scintigraphiques, le document FR.A.2 530 035 prévoit de disposer, dans un plan parallèle au plan de focalisation de l'organe observé, trois feuilles de substance luminescente munies chacune d'un collimateur orienté vers l'organe observé. Après exposition des feuilles au rayonnement de l'organe observé, on balaie ces feuilles avec des rayons excitateurs qui leur font émettre de la lumière, et on convertit la lumière en signaux électriques pour reproduire une image en utilisant le signal du plan de focalisation.

Pour l'enregistrement des images scintigraphiques, on peut utiliser aussi une camera à scintillations, ou gamma-camera. Cette gamma-camera est susceptible de tourner pas-à-pas autour d'un axe passant par l'organe à examiner, pour enregistrer des images scintigraphiques sous des incidences successives dont le nombre est en général 16, 32, 64 ou 128. A partir des informations recueillies pendant la rotation de la gamma-camera, un algorithme de reconstruction tomographique permet d'obtenir une série de coupes transverses de quelques millimètres d'épaisseur.

Avec une gamma-camera classique, on enregistre une seule image scintigraphique par incidence. Il en résulte que le principal inconvénient de la tosmoscintigraphie est la lenteur dans l'acquisition des informations.

Le but de l'invention est de réduire la durée globale d'acquisition des informations, en permettant la réalisation simultanée de plusieurs images scintigraphiques sous des incidences différentes à partir d'une position déterminée de la gamma-camera, ce qui permet de réduire le nombre de pas de rotation de la gamma-camera.

L'invention a pour objet un collimateur pour tomoscintigraphie, destiné à être placé devant le cristal sensible d'une camera à scintillations ou gamma-camera, susceptible de se déplacer en rotation pas-à-pas autour d'un axe passant par l'organe observé, afin d'enregistrer des images scintigraphiques sous des incidences successives du type comportant une pluralité de tubes parallèles adjacents destinés à être traversés dans leur longueur par le rayonnement en provenance de l'organe observé, constitué de plusieurs ensembles juxtaposés de tubes, la direction des tubes de chaque ensemble correspondant à celle du tube placé sensiblement au centre de l'ensemble et à celle du rayonnement en provenance de l'organe observé, de façon à définir pour chaque ensemble une incidence correspondant à une image scintigraphique de l'organe observé, de telle sorte que, à chaque pas de rotation de la gamma-camera, l'enregistrement d'informations corresponde à autant d'images qu'il y a d'ensembles dans le collimateur, caractérisé en ce que chaque ensemble latéral de tubes comporte deux grilles parallèles tenant les extrémités des tubes et susceptibles de se déplacer parallèlement l'une par rapport à l'autre, chacune des grilles étant commandée par un moteur électrique pour que la direction des tubes de l'ensemble soit toujours parallèle à la direction du rayonnement en provenance de l'organe observé, les moteurs électriques étant commandés par un microprocesseur en fonction de la distance entre la camera et l'organe observé.

A titre d'exemple et pour faciliter l'intelligence de la description, on a représenté au dessin annexé:

Figure 1 une de face d'un exemple de réalisation d'un collimateur selon l'invention.

Figure 2 une vue en coupe transversale du collimateur de la figure 1, dans deux positions correspondant à deux distances de mesure.

Le collimateur selon l'invention est destiné à être placé devant le cristal détecteur de la gamma-camera. Ce cristal est en général un monocristal, par exemple d'iodure de sodium dopé au thallium, qui scintille lorsqu'il reçoit le rayonnement en provenance de l'organe observé. Pour se limiter avec précision à ce rayonnement direct en éliminant les rayonnements diffusés on place devant le cristal un collimateur constitué d'une pluralité de tubes parallèles, adjacents, à section carrée par exemple, et dont les cloisons de séparation sont en plomb de préférence.

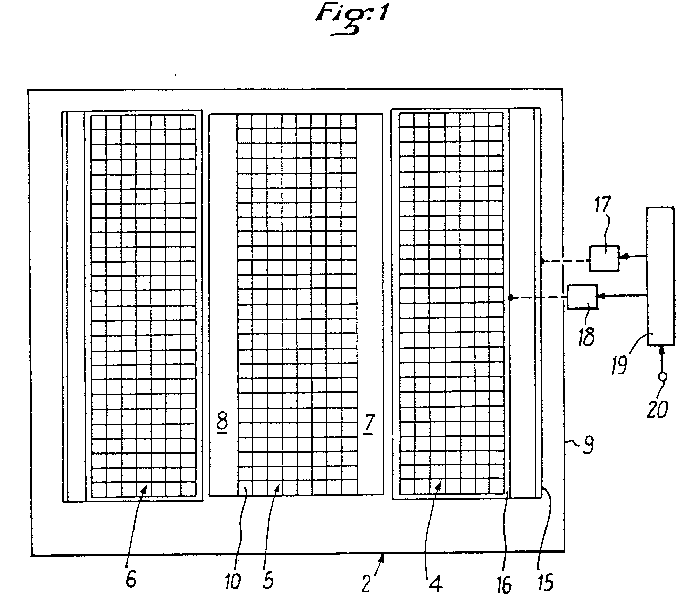

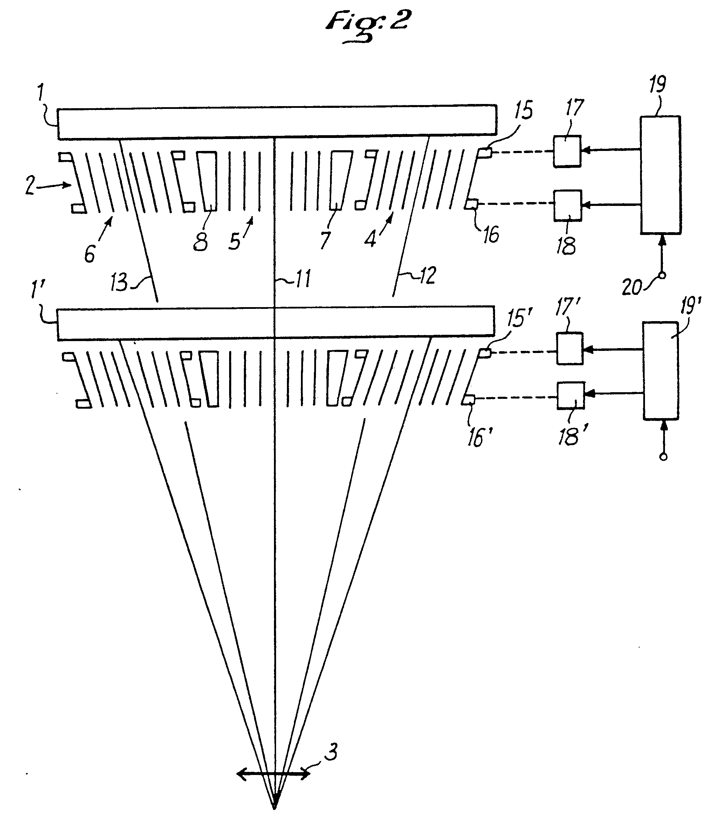

Selon l'invention, le cristal sensible de la gamma-camera est représenté en 1 (figure 2) et le collimateur en 2. L'organe observé est symbolisé en 3. Le collimateur 2 (figure 1) est constitué de trois ensembles 4, 5, 6, séparés par des intervalles opaques 7, 8. Chacun des ensembles 4, 5, 6, est constitué d'une pluralité de tubes parallèles. Ces tubes tels que 10 (figure 2), sont de section carrée dans l'exemple représenté. Leurs parois sont en plomb par exemple. La longueur des tubes est de l'ordre de 3 à 10 cm, leur largeur de l'ordre de 2 à 6 mm et l'épaisseur de leur paroi de séparation de l'ordre de 0,2 à 2 mm, dans l'exemple de réalisation décrit.

Pour chaque ensemble, les tubes sont tous parallèles. L'ensemble 5, qui est placé dans la zone centrale du cristal 1 est constitué de tubes dont la direction est perpendiculaire au plan du cristal, de façon à coïncider avec la direction du rayonnement 11 en provenance de l'organe observé.

Les tubes de l'ensemble 4 sont inclinés par rapport au cristal 1 de façon que leur direction coïncide avec celle du rayonnement 12. De même, la direction des tubes de l'ensemble 6 coïncide avec celle du rayonnement 13. Les rayonnements 12 et 13 font avec la direction du rayonnement 11 un angle bien défini. Cet angle qui correspond à une incidence bien définie, est choisi comme angle d'inclinaison des tubes de l'ensemble correspondant sur la perpendiculaire au cristal sensible.

Chaque ensemble de tubes 4, 5, 6 est disposé dans le collimateur de façon que le rayonnement correspondant, respectivement 12, 11, 13, aboutisse au tube placé sensiblement au milieu de l'ensemble. De cette manière, chaque ensemble de tubes permet la réalisation d'une image scintigraphique correspondant à une incidence déterminée.

Dans l'exemple représenté, le collimateur comprend trois ensembles, et pour chaque pas de rotation de la gamma-camera, celle-ci recueille trois images scintigraphiques de l'organe observé.

Ainsi, en s'adaptant sur une gamma-camera rotative existante, donc sans investissement lourd, le collimateur selon l'invention permet de réduire considérablement le nombre de pas de rotation pour l'acquisition du même nombre d'images scintigraphiques. Il en résulte une immobilisation moins longue de la gamma-camera pour chaque observation.

L'angle choisi pour les directions de rayonnement 12 et 13 par rapport à la direction de rayonnement 11 dépend du nombre total d'incidences désirées pour l'observation. La longueur des tubes, leur diamètre et l'épaisseur de leur cloison de séparation sont choisis en fonction des conditions d'expérimentation. Les paramètres à prendre en considération pour cette expérimentation sont en particulier: l'énergie du rayonnement émis par l'organe observé, le volume de cet organe et la résolution désirée pour l'image.

Avec une gamma-camera de type classique, on peut enregistrer des images scintigraphiques sous des incidences successives en procédant par rotation pas-à-pas autour de l'organe à observer. Cette rotation peut être accompagnée d'une variation de la distance de la camera à l'organe à observer. Dans ce cas, usuellement, la camera se déplace non pas sur un cercle mais sur une ellipse par exemple.

Sur la figure 2, on a représenté deux positions de la camera correspondant à deux distances différentes par rapport à l'organe observé toujours symbolisé en 3. Dans la position éloignée, le cristal porte la référence 1, et dans la position rapprochée, la référence 1. Pour assurer l'orientation correcte des ensembles de tubes 4 et 6 en fonction de la distance à l'objet à observer, ces ensembles sont montés sur des charpentes articulées, susceptibles de faire varier leur inclinaison, de manière que les tubes d'un ensemble 4, 6 soient toujours parallèles à la direction du rayonnement 12, 13 respectivement, passant par le milieu de l'ensemble de tubes correspondant. De cette façon, l'intersection des directions des ensembles de tubes se trouve toujours au centre de rotation de la camera.

Pour conserver la même qualité d'image au cours de la rotation elliptique de la camera, il est préférable que les images scintigraphiques se forment toujours à la même place sur le cristal. Il ne suffit donc pas de faire pivoter les tubes autour d'axes passant par leur milieu. Selon l'invention, les ensembles de tubes sont montés entre deux grilles 15, 16, placées au voisinage de leurs extrémités, l'une 6 vers l'organe à observer, l'autre 5 vers le cristal 1. Ces deux grilles sont disposées dans une charpente articulée et elles sont prévues pour se déplacer l'une par rapport à l'autre en restant parallèles entre elles et au cristal 1. Elles sont chacune commandées par un moteur électrique 17, 18, respectivement, qui assure leur déplacement en fonction de la distance entre l'organe à observer et la gamma-camera. Les deux moteurs 17, 18 ont une position fixe par rapport au cadre 9 de l'ensemble du collimateur. Ainsi les grilles 15, 16 sont déplacées par rapport à ce cadre pour que le point d'impact sur le cristal 1 des rayons moyens 12, 13 soit toujours à la même place. A titre d'exemple, la position de la gamma-camera la plus proche, ou la plus éloignée, de l'organe peut être fixée a priori, et la position correspondante des grilles et des ensembles de tubes, également. Les déplacements radiaux de la camera peuvent être mesurés par un capteur qui transmet l'information correspondante 20 à un microprocesseur 19 par exemple. Un logiciel permet la commande des moteurs électriques 17, 18 pour placer les tubes dans l'inclinaison souhaitée. Si la camera est elle-même commandée dans ses déplacements par un logiciel, c'est ce logiciel qui peut lui-même assurer la commande des moteurs 17, 18.

Avec la gamma-camera selon l'invention, on peut donc assurer une réduction du nombre d'étapes dans le processus d'acquisition des informations, en permettant même lorsque la distance de la camera à l'organe observé varie, la réalisation simultanée de plusieurs images scintigraphiques sous des incidences différentes à partir d'une position déterminée de la gamma-camera.

**Revendication**

1. Collimateur pour tomoscintigraphie,
– destiné à être placé devant le cristal sensible (1) d'une camera à scintillations ou gamma-camera susceptible de se déplacer en rotation pas-à-pas autour d'un axe passant par l'organe (3) observé, afin d'enregistrer des images scintigraphiques sous des incidences successives,
– du type comportant une pluralité de tubes (10) parallèles adjacents destinés à être traversés dans leur longueur par le rayonnement en provenance de l'organe (3) observé,
– constitué de plusieurs ensembles juxtaposés de tubes, la direction des tubes de chaque ensemble (4, 5, 6) correspondant à celle du tube placé sensiblement au centre de l'ensemble et à celle du rayonnement (12, 11, 13) en provenance de l'organe (3) observé, de façon à définir pour chaque ensemble (4, 5, 6) une incidence correspondant à une image scintigraphique de l'organe observé, de telle sorte que, à chaque pas de rotation de la gamma-camera, l'enregistrement d'informations corresponde à autant d'images qu'il y a d'ensembles dans le collimateur,
– caractérisé en ce que:
– chaque ensemble latéral de tubes (4, 6) comporte deux grilles (15, 16) parallèles tenant les extrémités des tubes et susceptibles de se déplacer parallèlement l'une par rapport à l'autre,

– chacune des grilles (1ə, 16) étant commandée par un moteur électrique (17, 18) pour que la direction des tubes de l'ensemble (4, 6) soit toujours parallèle à la direction du rayonnement (12, 13) en provenance de l'organe observé,

– les moteurs électriques (17, 18) étant commandés par un microprocesseur (19) en fonction de la distance entre la camera et l'organe observé.

## Patentanspruch

1. Kollimator für Tomoszintigraphie
– dazu bestimmt, vor dem sensiblen Kristall (1) einer Szintillations- oder Gammakamera aufgestellt zu werden, die geeignet ist, sich in schrittweise Rotationsbewegung um eine Achse zu bewegen, welche durch das beobachtete Organ (3) verläuft, um szintigraphische Bilder unter aufeinander folgenden Einfallswinkeln aufzuzeichnen.

– des Typs, der eine Anzahl von parallel nebeneinander liegenden Röhren (10) besitzt, die in ihrer Länge von der Strahlung, welche von dem beobachteten Organ (3) ausgeht, durchdrungen werden sollen,

– bestehend aus verschiedenen, nebeneinanderliegenden Röhreneinheiten, wobei die Richtung jeder dieser Einheiten (4, 5, 6) der Richtung der Röhre, welche genau in der Mitte der Einheit liegt, sowie der Richtung der Strahlung (12, 11, 13) entspricht, die von dem beobachteten Organ (3) ausgeht, sodaß man für jede Einheit (4, 5, 6) einen Einfallswinkel definieren kann, der einem szintigraphischen Bild des beobachteten Organs entspricht, sodaß bei jedem Rotationsschritt der Gammakamera die Aufzeichnung von Informationen so vielen Bildern entspricht, wie es Einheiten im Kollimator gibt,
– dadurch gekennzeichnet, daß:
– jede seitliche Röhreneinheit (4, 6) zwei parallele Gitter (15, 16) besitzt, die sich an den Röhrenenden befinden und geeignet sind, sich parallel zueinander zu bewegen,

– wobei jedes dieser Gitter von einem Elektromotor (15, 16) gesteuert wird, damit die Richtung der Röhren der Einheit (4, 6) stets parallel zur Richtung der Strahlung (12, 13) ist, die von dem beobachteten Organ ausgeht,
– wobei die Elektromotoren (17, 18) von einem Mikroprozessor (19) in Abhängigkeit von der Entfernung zwischen der Kamera und dem beobachteten Organ gesteuert werden.

## Claim

1. A collimator for tomoscintigraphy,
– to be placed in front of the sensitive crystal (1) of a scintillation camera or gamma camera which is capable of rotating step by step about an axis passing through the element (3) observed, in order to record scintigraphic images at successive incidences,
– of the type comprising a plurality of adjacent parallel tubes (10) to be traversed in their longitudinal direction by the ray coming from the element (3) observed,
– formed of several juxtaposed groups of tubes, the direction of the tubes of each group (4, 5, 6) corresponding to that of the tube placed approximately at the centre of the group and to that of the ray (12, 11, 13) coming from the element (3) observed, so as to define for each group (4, 5, 6) an incidence corresponding to a scintigraphic image of the element observed, such that, at each step of rotation of the gamma camera, the recording of information corresponds to as many images as there are groups in the collimator,
– characterised in that:
– each lateral group of tubes (4, 6) comprises two parallel grids (15, 16) holding the ends of the tubes and capable of moving parallel to one another,
– each of said grids (15, 16) being controlled by an electric motor (17, 18) so that the direction of the tubes of the group (4, 6) is always parallel to the direction of the ray (12, 13) coming from the element observed,
– the electric motor (17, 18) being controlled by a microprocessor (19) according to the distance between the camera and the element observed.

# Fig:1

## Fig. 2